# EUROPEAN PATENT SPECIFICATION

(11) **EP 0 422 029 B1**
(45) Date of publication and mention of the grant of the patent: **12.04.1995**
(21) Application number: 89906414.1
(22) Date of filing: 07.06.1989
(51) Int. Cl.: A61B 5/04

(54) **LOW RESISTANCE ELECTRICAL PICK-UP**
ELEKTRISCHER SENSOR MIT EINEM NIEDRIGEN WIDERSTAND
CAPTEUR ELECTRIQUE A BASSE RESISTANCE

(30) Priority: 08.06.1988 GB 8813570
(43) Date of publication of application: 17.04.1991
(73) Proprietor: MONITORING TECHNOLOGY LIMITED, London SE1 9RT (GB)
(72) Inventor: BAND, David, Marston, Surrey KT6 6RH (GB); PENMAN, David, Gerard, London SE11 6SS (GB)
(74) Representative: Hulse, Thomas Arnold
(86) International application number: PCT/GB89/00631
(87) International publication number: WO 89/11819

(56) References cited:
- US-A- 4 589 418
- Medical Progress through Technology, vol. 9, nos. 2/3, 1982, Springer Verlag (Berlin, DE), M.R. Neuman: "Physical and chemical sensors for medical instrumentation", pages 95-104
- IEEE Transactions on Biomedical Engineering, vol. BME-24, no. 2, March 1977, (New York, US), G.E. Loeb et al.: "Parylene as a chronically stable, reproducible microelectrode insulator", pages 121-128
- Obstetrics and Gynecology, vol. 30, no. 2, August 1967, E.H. Hon: "Instrument and Method. Instrumentation of fetal heart rate and fetal electrocardiography", pages 281-286

## Description

This invention relates to a low resistance electrical pickup - more particularly - but not exclusively - for use in a fetal scalp electrode or other biosensing electrode, and the manufacture thereof.

An article in Obstetrics and Gynaecology, Vol. 30, No. 2, August 1967 entitled "Instrument & Method: Instrumentation of Fetal Heart Rate and Fetal Electrocardiography: III Fetal ECG Electrodes: Further Observations" describes and illustrates an electrode assembly including (1) a 18-mm nickel-silver wound clip covered by heat-shrinkable tubing used to support (2) a negative electrode comprising two 5 mm bent lengths of fine silver wire soldered to the ends of a 25-mm length of green hook-up wire, and an additional 50-cm length of green hook-up wire also soldered to one of the silver wires, and with a 15-mm length of heat-shrinkable tubing shrunk in place, the clip also being used to support (3) a positive electrode comprising a 50-cm length of red hook-up wire soldered to a coil of silver wire with the soldered joint covered with epoxy cement and heat-shrinkable tubing shrunk over the joint; the completed electrode assembly is dipped in insulating varnish and, after draining and baking, the insulating varnish is removed from the tips of the negative electrode and a portion of the positive electrode spiral, and finally the electrodes are lightly chloridized.

According to the present invention, a method of manufacture of a low resistance electrical pick-up is characterised by applying to a length of stainless steel wire a first coating layer of nickel, and a subsequent coating layer of silver, optionally with an intervening layer of copper, an area of the silver layer then being chloridized and coated with bio-compatible plastics material. A further length or lengths of non-chloridized silver layer may be coated with a non-conducting varnish.

In a particular application of the invention to a fetal scalp electrode or other biosensing electrode of the type having an arcuate needle of stainless steel - which may serve as a reference electrode and/or measuring electrode and/or introducer for a separate electrode - the arcuate needle is plated with nickel and then silver, optionally with an intervening layer of copper, and an area of the silver layer at least on the outside of the curve of the needle, and possibly spaced from the point of the needle, is chloridized and then coated with bio-compatible plastics, and the remainder of the silver layer is coated with a non-conducting varnish.

Also in accordance with the present invention, a low resistance electrical pick-up is characterised by a length of stainless steel wire, a first layer of nickel, a subsequent layer of silver, optionally with an intervening layer of copper, a chloridized area of the silver layer, and a coating of bio-compatible plastics material. A further length or further lengths of non-chloridized silver layer may have a coating of a non-conducting varnish.

In the particular application of the invention to a fetal scalp electrode or other biosensing electrode of the type having an arcuate needle of stainless steel, the electrode is characterised in that the arcuate needle has a first layer of nickel, a subsequent layer of silver, optionally with an intervening layer of copper, a chloridized area of the silver layer at least on the outside of the curve of the needle, a coating of bio-compatible plastics material over the chloridized area, and a coating of a non-conducting varnish over the remainder of the silver layer.

The bio-compatible plastics material may be "Tecoflex" (Registered Trade Mark), a bio-compatible polyurethane resin. The chloridized silver area can be given a thin coating of "Tecoflex" by dipping it into a solution of "Tecoflex" in tetrahydrofuran (THF) which is allowed to set (approximately 12 to 24 hours) before the remaining areas of the electrode are painted with a non-conducting varnish.

A number of embodiments of the invention will now be described by way of example only, with reference to the accompanying diagrammatic drawings, in which:-
Figure 1 is a fragmentary enlarged side elevation of a basic low resistance electrical pick-up manufactured in accordance with the invention;
Figure 2 is an even greater enlarged cross-section on the line II-II of Figure 1;
Figure 3 corresponds to Figure 2 but includes a slight modification;
Figure 4 is a greatly enlarged skeletal perspective view of the head member of a "Surgicraft-Copeland" fetal scalp electrode (FSE) including a low resistance electrical pick-up manufactured in accordance with the invention;
Figure 5 is a fragmentary ECG trace obtained using a FSE as in Figure 4; and
Figure 6 is a fragmentary ECG trace obtained with an un-modified "Surgicraft-Copeland" FSE for comparison with the trace of Figure 5, the paper speed in Figure 5 being twice that in Figure 6.

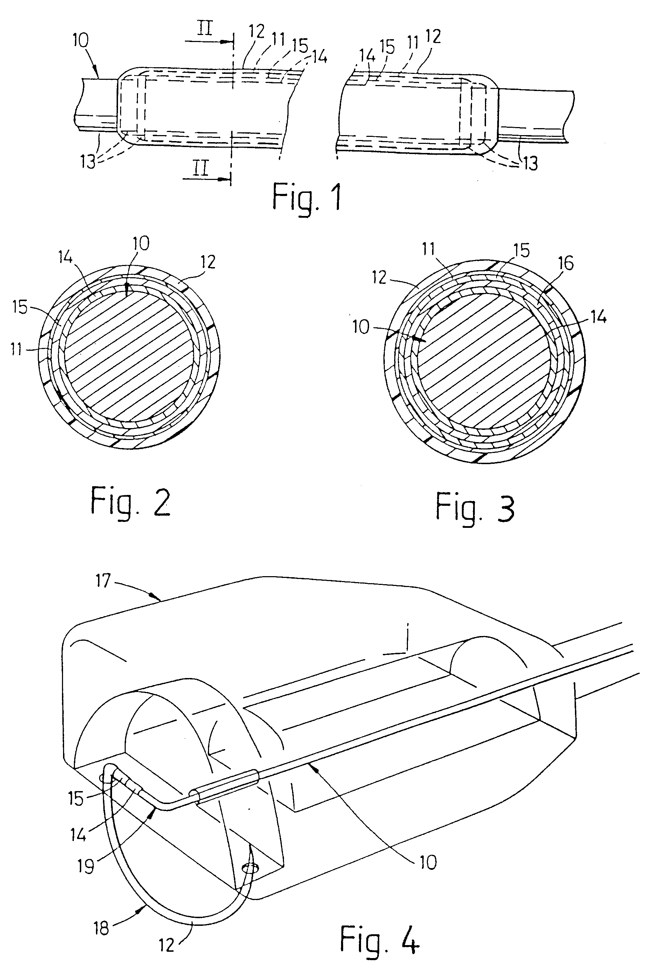

The thickness of the coating layers in Figures 1 to 3 have been greatly exaggerated, even compared to the enlarged scale, for the sake of clarity.

In Figures 1 and 2, a low resistance electrical pick-up comprises a length of stainless steel wire 10̸ having a first coating layer 14 of nickel, and a subsequent coating layer 15 of silver, an area (or length) 11 of the silver layer being chloridised (indicated in Figure 1 by the thicker broken line) and then coated with bio-compatible plastics material 12, e.g., "Tecoflex". Further lengths 13 of non-chloridized silver layer 15, and preferably also adjacent exposed lengths of the nickel layer 14 and stainless steel wire 10̸ may be coated with a non-conducting varnish.

Figure 3 corresponds closely to Figure 2 but indicates a layer 16 of copper between the nickel layer 14 and the silver layer 15.

In Figure 4, the head member 17 of a "surgicraft-Copeland" FSE, as described in GB-PS 1 316 0̸72 or GB-PS 1 523 263 (or US-PS 4 151 835), has an arcuate needle 18 formed on the outer end of a radial arm 19 from a main stainless steel wire 10̸, the arcuate needle 18 being plated with nickel 14 and then silver 15 (optionally with an intervening copper layer, not shown, but refer to Figure 3), an area (not shown) of the silver layer at least on the outside of the curve of the needle 18, and possibly spaced from the point of the needle, is chloridized and then coated with bio-compatible plastics 12, and the remainder of the silver layer is coated with a non-conducting varnish.

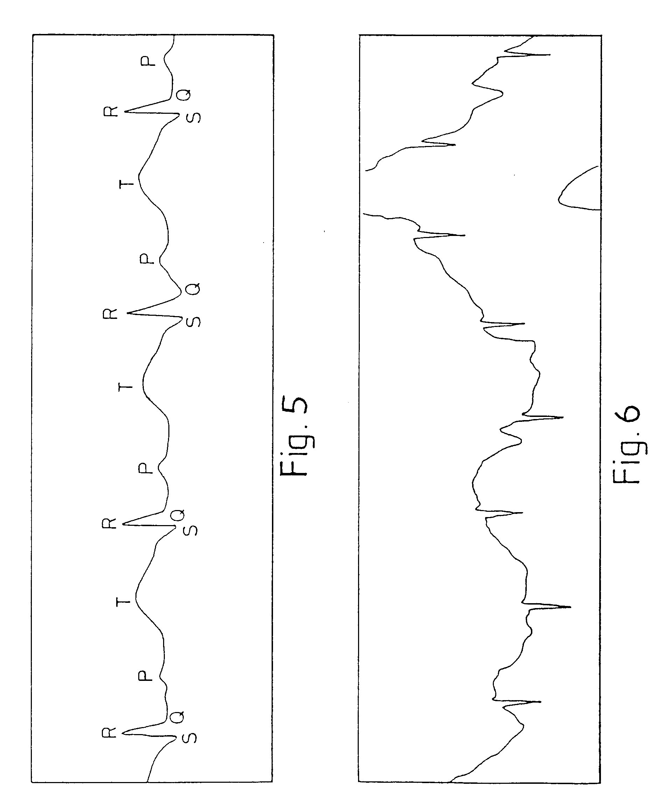

The fundamental advantage of the low resistance "Surgicraft-Copeland" FSE of Figure 4 can be appreciated by comparing Figures 5 and 6. The bare stainless steel arcuate needle electrode of the unmodified "Surgicraft-Copeland" FSE is highly resistant to low frequency electrical activity, so the trace of Figure 6 is erratic, and it also shows enormous variability because of the added effect of feto-maternal movement. In contrast, the trace of Figure 5 shows that low frequency electrical activity from the fetus can be picked up with great accuracy by the low resistance "Surgicraft-Copeland" FSE of Figure 4, and consistent waveforms (P,QRS and T waves) of the cardiac cycle can be more easily identified. This allows more complex fetal ECG waveform analysis and reduces the need for extensive signal processing and averaging, as has been necessary with the unmodified "Surgicraft-Copeland" FSE.

The invention is also applicable to other types of fetal scalp electrodes, such as the spiral electrodes of Corometrics Medical systems Inc. US Re 28990̸, the clip-like electrodes of T.C. Neward EP 0̸ 0̸0̸7 70̸2, and the outwardly splayed electrodes of Kontron Inc. EP 0̸ 0̸99 0̸77, or to other electrodes or probes, such as the outwardly splayed anchor needles in the cardiac probe of W. Mohl EP 0̸ 0̸0̸4 967.

## Claims

1. A method of manufacture of a low resistance electrical pick-up characterized by applying to a length of stainless steel wire (10) a first coating layer (14) of nickel, and a subsequent coating layer (15) of silver, an area (11) of the silver layer then being chloridized and coated with bio-compatible plastics material (12).

2. A method as in Claim 1, characterised in that the bio-compatible plastics material (12) is a bio-compatible polyurethane resin known under the Registered Trade Mark "Tecoflex".

3. A method as in Claim 1 or Claim 2, characterised in that an intervening layer (16) of copper is provided between the nickel and silver layers (14, 15).

4. A method as in any one of Claims 1 to 3, characterised in that any further length or lengths (13) of non-chloridized silver layer (15) is/are coated with a non-conducting varnish.

5. A method of manufacture of a fetal scalp electrode or other biosensing electrode of the type having an arcuate needle (18) of stainless steel, characterised in that the arcuate needle is plated with nickel (14) and then silver (15), and an area (11) of the silver layer, at least on the outside of the curve of the needle, is then chloridized and coated with bio-compatible plastics (12), and the remainder (13) of the silver layer (15) is coated with a non-conducting varnish.

6. A method as in Claim 5, characterised in that the bio-compatible plastics is a bio-compatible polyurethane resin known under the Registered Trade Mark "Tecoflex".

7. A method as in Claim 5 or Claim 6, characterised in that an intervening layer (16) of copper is provided between the nickel and silver layers (14, 15).

8. A low resistance electrical pick-up characterised by a length of stainless steel wire (10), a first layer (14) of nickel, a subsequent layer (15) of silver, a chloridized area (11) of the silver layer, and a coating (12) of bio-compatible plastics material over the chloridized silver area.

9. A low resistance electrical pick-up as in Claim 8, characterised in that the bio-compatible plastics material (12) is a bio-compatible polyurethane resin known under the Registered Trade Mark "Tecoflex".

10. A low resistance electrical pick-up as in Claim 8 or Claim 9, characterised in that there is an intervening layer (16) of copper between the nickel and silver layers (14, 15).

11. A low resistance electrical pick-up as in any one of Claims 8 to 10, characterised in that a further length or further lengths (13) of non-chloridized silver layer (15) has/have a coating of a non-conducting varnish.

12. A fetal scalp electrode or other biosensing electrode of the type having an arcuate needle (18) of stainless steel, characterised in that the arcuate needle has a first layer (14) of nickel, a subsequent layer (15) of silver, a chloridized area (11) of the silver layer at least on the outside of the curve of the needle, a coating (12) of bio-compatible plastics material over the chloridized area, and a coating of a non-conducting varnish over the remainder (13) of the silver layer (15).

13. An electrode as in Claim 12, characterised in that the bio-compatible plastics material (12) is a bio-compatible polyurethane resin known under the Registered Trade Mark "Tecoflex".

14. An electrode as in Claim 12 or Claim 13, characterised in that there is an intervening layer (16) of copper between the nickel and silver layers (14, 15).

## Patentansprüche

1. Methode zur Herstellung einer elektrischen Aufnahmeeinrichtung mit geringem Widerstand, **dadurch gekennzeichnet,** daß auf einem Edelstahldrahtabschnitt (10) eine erste Nickelbeschichtung (14) und eine zweite Silberbeschichtung (15) aufgebracht werden, wobei ein Bereich (11) der Silberschicht dann chloriert und mit einem bioverträglichen Kunststoffmaterial (12) beschichtet wird.

2. Methode nach Anspruch 1, **dadurch gekennzeichnet,** daß das bioverträgliche Kunststoffmaterial (12) ein bioverträgliches Polyurethanharz ist, das unter der eingetragenen Handelsmarke "Tecoflex" bekannt ist.

3. Methode nach Anspruch 1 oder Anspruch 2, **dadurch gekennzeichnet,** daß zwischen der Nickel- und Silberbeschichtung (14, 15) eine Zwischenschicht (16) aus Kupfer angeordnet ist.

4. Methode nach einem der Ansprüche 1 bis 3, **dadurch gekennzeichnet,** daß ein weiterer Abschnitt oder weitere Abschnitte (13) der nicht chlorierten Silberbeschichtung (15) mit einem nichtleitenden Lack beschichtet ist/sind.

5. Methode zur Herstellung einer fötalen Kopfhautelektrode oder einer anderen Bioelektrode, deren Ausführung **dadurch gekennzeichnet** ist, daß sie eine gebogene Nadel (18) aus Edelstahl aufweist, weiterhin **dadurch gekennzeichnet,** daß diese gebogene Nadel zunächst mit Nickel (14) und dann mit Silber (15) beschichtet ist und ein Bereich (11) der Silberbeschichtung, zumindest im Außenbereich der Nadelkrümmung, chloriert und mit einem bioverträglichen Kunststoff (12) beschichtet wird, während der verbleibende Teil (13) der Silberbeschichtung (15) mit einem nichtleitenden Lack überzogen wird.

6. Methode nach Anspruch 5, **dadurch gekennzeichnet,** daß das bioverträgliche Kunststoffmaterial ein bioverträgliches Polyurethanharz ist, das unter der eingetragenen Handelsmarke "Tecoflex" bekannt ist.

7. Methode nach Anspruch 5 oder 6, **dadurch gekennzeichnet,** daß zwischen der Nickel- und Silberbeschichtung (14, 15) eine Zwischenschicht (16) aus Kupfer angeordnet ist.

8. Elektrische Aufnahmeeinrichtung mit geringem Widerstand, **gekennzeichnet** durch einen Edelstahldrahtabschnitt (10), eine erste Nickelbeschichtung (14), eine zweite Silberbeschichtung (15), einen chlorierten Bereich (11) der Silberschicht und ein bioverträgliches Kunststoffmaterial (12), das auf den chlorierten Silberbereich aufgetragen wird.

9. Elektrische Aufnahmeeinrichtung mit geringem Widerstand nach Anspruch 8, **dadurch gekennzeichnet,** daß das bioverträgliche Kunststoffmaterial (12) ein bioverträgliches Polyurethanharz ist, das unter der eingetragenen Handelsmarke "Tecoflex" bekannt ist.

10. Elektrische Aufnahmeeinrichtung mit geringem Widerstand nach Anspruch 8 oder 9, **dadurch gekennzeichnet,** daß zwischen der Nickel- und Silberbeschichtung (14, 15) eine Zwischenschicht (16) aus Kupfer angeordnet ist.

11. Elektrische Aufnahmeeinrichtung mit geringem Widerstand nach einem der Ansprüche 8 bis 10, **dadurch gekennzeichnet,** daß ein weiterer Abschnitt oder weitere Abschnitte (13) der nicht chlorierten Silberbeschichtung (15) mit einem nichtleitenden Lack beschichtet ist/sind.

12. Fötale Kopfhautelektrode oder eine andere Bioelektrode, deren Ausführung **dadurch gekennzeichnet** ist, daß sie eine gebogene Nadel (18) aus Edelstahl aufweist, gekennzeichnet weiterhin dadurch, daß diese gebogene Nadel zunächst mit Nickel (14) und dann mit Silber (15) beschichtet ist und ein Bereich (11) der Silberbeschichtung, zumindest im Außenbereich der Nadelkrümmung, chloriert und mit einem bioverträglichen Kunststoff (12) beschichtet wird, während der verbleibende Teil (13) der Silberbeschichtung (15) mit einem nichtleitenden Lack überzogen wird.

13. Elektrode nach Anspruch 12, **dadurch gekennzeichnet,** daß das bioverträgliche Kunststoffmaterial (12) ein bioverträgliches Polyurethanharz ist, das unter der eingetragenen Handelsmarke "Tecoflex" bekannt ist.

14. Elektrode nach Anspruch 12 oder 13, **dadurch gekennzeichnet,** daß zwischen der Nickel- und Silberbeschichtung (14, 15) eine Zwischenschicht (16) aus Kupfer angeordnet ist.

## Revendications

1. Procédé de fabrication d'un capteur à faible résistance électrique, caractérisé par le fait d'appliquer à une longueur de fil en acier inoxydable (10) une première couche de revêtement (14) de nickel, et une couche de revêtement subséquente (15) d'argent, une zone de la couche d'argent étant alors chlorée et revêtue avec une matière plastique biocompatible (12).

2. Procédé selon la revendication 1, caractérisé en ce que la matière plastique biocompatible (12) est une résine polyuréthanne biocompatible connue sous la marque déposée "Tecoflex".

3. Procédé selon la revendication 1 ou la revendication 2, caractérisé en ce qu'une couche intermédiaire (16) de cuivre est prévue entre les couches de nickel et d'argent (14, 15).

4. Procédé selon l'une quelconque des revendications 1 à 3, caractérisé en ce que toute(s) longueur(s) supplémentaire (13) de couche d'argent non chlorée (15) est/sont revêtue(s) avec un vernis non-conducteur.

5. Procédé de fabrication d'une électrode crânienne foetale ou autre électrode de bio-captage du type ayant une aiguille arquée (18) en acier inoxydable, caractérisé en ce que l'aiguille arquée est plaquée avec du nickel (14) et ensuite de l'argent (15), et une zone (11) de la couche d'argent, au moins à l'extérieur de la courbure de l'aiguille, est alors chlorée et revêtue de matière plastique biocompatible (12), et le restant (13) de la couche d'argent (15) est revêtue avec un vernis non-conducteur.

6. Procédé selon la revendication 5, caractérisé en ce que la matière plastique biocompatible est une résine polyuréthanne bio-compatible connue sous la marque déposée "Tecoflex".

7. Procédé selon la revendication 5 ou la revendication 6, caractérisé en ce qu'une couche intermédiaire (16) de cuivre est prévue entre les couches de nickel et d'argent (14, 15).

8. Capteur à faible résistance électrique, caractérisé par une longueur de fil d'acier inoxydable (10), une première couche (14) de nickel, une couche subséquente (15) d'argent, une zone chlorée (11) de la couche d'argent, et un revêtement (12) en matière plastique biocompatible sur la zone d'argent chlorée.

9. Capteur à faible résistance électrique selon la revendication 8, caractérisé en ce que la matière plastique biocompatible (12) est une résine polyuréthanne biocompatible connue sous la marque déposée "Tecoflex".

10. Capteur à faible résistance électrique selon la revendication 8 ou la revendication 9, caractérisé en ce qu'une couche intermédiaire (16) de cuivre est prévue entre les couches de nickel et d'argent (14, 15).

11. Capteur à faible résistance électrique selon l'une quelconque des revendications 8 à 10, caractérisé en ce que toute(s) longueur(s) supplémentaire (13) de couche d'argent non chlorée (15) est/sont revêtue(s) avec un vernis non-conducteur.

12. Electrode crânienne foetale ou autre électrode de bio-captage du type ayant une aiguille arquée (18) en acier inoxydable, caractérisée en ce que l'aiguille arquée a une première couche (14) de nickel, une couche subséquente (15) d'argent, une zone chlorée (11) de la couche d'argent au moins à l'extérieur de la courbure de l'aiguille, un revêtement (12) de matière plastique biocompatible sur la zone chlorée, et un revêtement en vernis non-conducteur sur le restant (13) de la couche d'argent (15).

13. Electrode selon la revendication 12, caractérisée en ce que la matière plastique biocompatible (12) est une résine polyuréthanne biocompatible connue sous la marque déposée "Tecoflex".

14. Electrode selon la revendication 12 ou la revendication 13, caractérisée en ce qu'une couche intermédiaire (16) de cuivre est prévue entre les couches de nickel et d'argent (14, 15).
